# EUROPÄISCHE PATENTSCHRIFT

(11) **EP 4 349 400 B1**
(45) Veröffentlichungstag und Bekanntmachung des Hinweises auf die Patenterteilung: **22.01.2025**
(21) Anmeldenummer: 22200325.3
(22) Anmeldetag: 07.10.2022
(51) Int. Cl.: A61N 5/06

(54) **WÄRMEBEHANDLUNGSVORRICHTUNG**
HEAT TREATMENT APPARATUS
DISPOSITIF DE TRAITEMENT THERMIQUE

(43) Veröffentlichungstag der Anmeldung: 10.04.2024
(73) Patentinhaber: Mattheiss, Gerd, 7203 Trimmis (CH)
(72) Erfinder: Pecher, Otto, 85653 Aying b. München (DE); Zeiger, Thomas, 6134 Vomp (AT); Mattheiss, Gerd, 7203 Trimmis (CH)
(74) Vertreter: Fuchs Patentanwälte Partnerschaft mbB

(56) Entgegenhaltungen:
- EP-A2- 2 168 632
- CN-A- 106 913 959
- DE-A1- 102014 106 497
- US-A1- 2012 010 684

## Beschreibung

Die Anmeldung betrifft eine Wärmebehandlungsvorrichtung gemäß dem Oberbegriff von Anspruch 1. Die Anmeldung betrifft außerdem eine Wärmekabine und ein Verfahren.

### Technischer Hintergrund

Wärmebehandlungsvorrichtungen sind bekannt. Sie werden üblicherweise dazu verwendet, Wärme in Form von Wärme- bzw. Infrarotstrahlung auf den Körper oder bestimmte Körperregionen eines Benutzers aufzubringen. Eine Wärmebehandlung geschieht oft als therapeutische oder therapiebegleitende Maßnahme, etwa im Rahmen physiotherapeutischer Behandlungen, oder allgemein zur Förderung des Wohlbefindens.

Der menschliche Körper verfügt nicht über ein absolutes Temperaturempfinden. Zudem werden im Zusammenhang mit Wärmebehandlungen selbst Temperaturen, die bereits zu einer Schädigung der Haut durch übermäßige Erwärmung führen können, von vielen Benutzern oft nicht als unangenehm heiß empfunden. Darüber hinaus ist eine geeignete Temperatursteuerung in Wärmebehandlungsvorrichtungen dadurch erschwert, dass die Oberflächentemperatur in einem wärmebestrahlten Hautbereich stark vom Abstand des Benutzers von der Wärmequelle abhängt. Bei installierten Wärmebehandlungsvorrichtungen, etwa in Wärmekabinen oder bei Verwendung einer Decken- oder Wandmontierung, variiert dieser Abstand oft in Abhängigkeit von der Körpergröße oder der bestrahlten Körperregion. Zudem hängt die Oberflächentemperatur von einer Wärmeaufnahme- und Wärmeverarbeitungsfähigkeit der Haut abhängig von der Hautdurchblutung und einem Wärmeabbauvermögen des jeweiligen Benutzers ab. Das Wärmeabbauvermögen ist hauptsächlich durch einen Wärmetransport von der Haut in tieferliegende Regionen des Körpers infolge der Blutzirkulation bestimmt und ist von Benutzer zu Benutzer verschieden.

Um einerseits eine hinreichende Erwärmung der Hautoberfläche für eine beabsichtigte Wärmebehandlung zu erzielen, aber gleichzeitig Verletzungen der Haut infolge von zu starker und/oder zu lange anhaltender Wärmestrahlung zu vermeiden, ist es bekannt, eine Hauttemperatur in einem bestrahlten Hautbereich während einer Wärmebehandlung wiederholt mittels Sensoren zu erfassen. Die erfasste Hauttemperatur wird anschließend automatisch mit einer Bezugsgröße, bei der es sich beispielsweise um eine gespeicherte obere Schwellenwerttemperatur handelt, verglichen. Ergibt der Vergleich, dass die erfasste Hauttemperatur größer ist als die obere Schwellenwerttemperatur, wird eine Heizleistung der Wärmebehandlungsvorrichtung automatisch verringert. Beispielsweise wird die Heizleistung für einen bestimmten Zeitraum und/oder um einen bestimmten Betrag verringert, sodass ein weiteres Aufwärmen der Hautregion vermieden wird bzw. ein Abklingen der Überwärmung der Hautregion erfolgen kann.

Bei einigen solcher Wärmebehandlungsvorrichtungen ist zudem bekannt, eine Heizleistung nicht zu verringern oder gegebenenfalls sogar zu erhöhen, wenn die erfasste Hauttemperatur in dem bestrahlten Hautbereich unterhalb einer unteren Schwellenwerttemperatur, die niedriger als die obere Schwellenwerttemperatur ist, liegt. Dies gestattet eine Regelung der Heizleistung gemäß einer Solltemperatur. Insbesondere ist so ermöglicht, die Hauttemperatur im bestrahlten Hautbereich benutzerunabhängig über einen längeren Zeitraum stabil innerhalb eines vorgegebenen Bereichs um eine Solltemperatur, die für die Wärmebehandlung vorgesehen ist, zu halten.

Bei bekannten Wärmebehandlungsvorrichtungen, bei denen eine Heizleistung wie voranstehend beschrieben anhand einer erfassten Hauttemperatur innerhalb eines Bereichs um eine Solltemperatur regelbar ist, werden Keramikwärmestrahler eingesetzt. In einigen Keramikwärmestrahlern wird ein elektrischer Heizstrom durch ein Heizelement aus Keramik geleitet. Ein elektrischer Widerstand der Keramik führt zur Umwandlung eines Teils des angelegten Heizstroms in Wärme, von der wiederum ein Teil in Form von Infrarotstrahlung von dem Keramikelement abgestrahlt wird. Bei anderen Keramikwärmestrahlern umgibt eine Keramikhülle einen Heizdraht aus Metall. Mittels des Heizdrahts wird die Keramikhülle von innen aufgeheizt und gibt die Wärme nach außen ab. Die Keramikhülle begünstigt eine gleichmäßige Wärmeabstrahlung an einer vergrößerten Abstrahlfläche. Um die Wärmeleitung zwischen Heizdraht und Keramikhülle zu erhöhen, ist in einigen Fällen ein Zwischenraum mit Quarz- oder sogenanntem Lavasand gefüllt. Keramikwärmestrahler sind zudem durch ein verhältnismäßig stabiles Wärmeabstrahlungsverhalten auch bei Fluktuationen des Heizstroms und auch bei größerer räumlicher Erstreckung des Heizelements, wie sie Keramikelemente üblicherweise aufweisen, gekennzeichnet.

Der Infrarotstrahler gemäß der DE 10 2014 106497 weist durch seine Steuerung nicht einen Betriebszustand wie beansprucht aus.

Ausgehend davon besteht eine Aufgabe darin, eine Wärmebehandlungsvorrichtung mit erweiterten Anwendungsmöglichkeiten und/oder verbesserter Verletzungssicherheit bereitzustellen.

### Abriss der Erfindung

Die Aufgabe wird durch eine Wärmebehandlungsvorrichtung mit den Merkmalen nach Anspruch 1, eine Wärmekabine nach Anspruch 13 und ein Verfahren nach Anspruch 15 gelöst. Demnach umfasst gemäß einem Aspekt eine Wärmebehandlungsvorrichtung wenigstens einen Infrarotstrahler, der wenigstens eine steuerbare Wärmequelle umfasst und dazu angeordnet ist, Infrarotstrahlung, die mittels der Wärmequelle erzeugt ist, an einen Hautbereich eines Benutzers der Wärmebehandlungsvorrichtung auszusenden. Die Wärmebehandlungsvorrichtung umfasst außerdem wenigstens einen Wärmesensor, der dazu angeordnet ist, eine Oberflächentemperatur in dem Hautbereich des Benutzers zu erfassen, und eine Steuerungseinrichtung, die dazu ausgebildet ist, eine Heizleistung des wenigstens einen Infrarotstrahlers wenigstens teilweise auf der Grundlage der erfassten Oberflächentemperatur in dem Hautbereich des Benutzers zu steuern. Der Infrarotstrahler weist in einem Betriebszustand, der einer an den Benutzer abgegebenen Wärmeleistung im Bereich von 20 bis 120 Milliwatt pro Quadratzentimeter in dem Hautbereich des Benutzers entspricht, eine minimale Verzögerung von weniger als 5 Sekunden zum Erzielen einer Differenz von wenigstens 20% in der an den Benutzer in dem Hautbereich abgegebenen Wärmeleistung auf.

Eine erfindungsgemäße Wärmebehandlungsvorrichtung gestattet eine verbesserte Bestimmung einer Wärmeabbaurate des Benutzers. Das Bestimmen der Wärmeabbaurate kann dabei insbesondere mittels kurzzeitigen Erwärmens eines Hautbereichs des Benutzers und/oder mittels kurzfristiger Veränderungen einer an den Benutzer in dem Hautbereich abgegebenen Wärmeleistung erfolgen. Eine genauere und sicherere Steuerung der Heizleistung des Infrarotstrahlers während einer Wärmebehandlung gemäß einer Wärmeabbaurate des Benutzers ist so ermöglicht.

Eine derartige Wärmebehandlungsvorrichtung gestattet außerdem ein verbessertes Anpassen einer an den Benutzer abgegebenen Wärmeleistung in Abhängigkeit von einer, insbesondere kontinuierlich, erfassten Oberflächentemperatur in dem Hautbereich des Benutzers im Fall von sich ändernden äußeren Bedingungen während einer Wärmebehandlung. Dazu zählen beispielsweise Körperbewegungen des Benutzers, durch die ein Abstand des Benutzers von dem wenigstens einen Infrarotstrahler wesentlich verändert wird. Eine Verletzungsgefahr infolge von Überwärmung ist so verringerbar. Zudem ist das Auftreten von zu niedriger Wärmeleistung in solchen Fällen verringerbar. Eine Wirksamkeit der Wärmebehandlung ist dadurch verbessert.

Die Wärmebehandlungsvorrichtung gestattet außerdem eine Erweiterung durchführbarer Wärmebehandlungen. Insbesondere sind Wärmebehandlungen gemäß einem vorgesehenen zeitlich veränderlichen Profil der Oberflächentemperatur in dem Hautbereich des Benutzers, die eine Änderung der an den Benutzer abgegebenen Wärmeleistung in einem verhältnismäßig kurzen Zeitraum vorsehen, begünstigt. Dazu zählen Reizbehandlungen. Dies ist dabei zudem ohne eine Beeinträchtigung einer Zuverlässigkeit der Wärmeregulierung ermöglicht.

Die Wärmebehandlungsvorrichtung gestattet ein Verändern der an den Benutzer in dem Hautbereich abgegebenen Wärmeleistung mit einer Verzögerung die geringer ist, als es zum Einstellen eines thermodynamischen Gleichgewichts zwischen einer an den Benutzer dabei abgegebenen Wärmeleistung und einer sich infolge des Veränderns der Wärmeleistung gleichzeitig verändernden Oberflächentemperatur in dem Hautbereich erforderlich ist. Das begünstigt Wärmereizbehandlungen, insbesondere durch kurzfristiges Erhöhen und/oder Verringern der abgegebenen Wärmeleistung. Zudem begünstigt es ein Kalibrieren der Wärmebehandlungsvorrichtung gemäß einem benutzerspezifischen Wärmeabbauvermögen durch kurzfristiges Erhöhen und/oder Verringern der abgegebenen Wärmeleistung, wie nachstehend näher beschrieben.

Ebenso gestattet die Wärmebehandlungsvorrichtung die Durchführung von Wärmebehandlungen, die eine zeitlich konstante oder wenig veränderliche Abgabe von Wärmeleistung über einen längeren Zeitraum vorsehen. Dazu können Durchwärmungsanwendungen zählen, die auf eine Durchwärmung größerer Körperregionen einschließlich des gesamten Körpers des Benutzers gerichtet sind, insbesondere bei einer Verwendung der Wärmebehandlungsvorrichtung in einer Wärmekabine.

Die Steuerungseinrichtung kann dazu ausgebildet sein, die Heizleistung des Infrarotstrahlers, insbesondere vollständig, durch Steuern eines Heizstroms des Infrarotstrahlers zu steuern. Der Infrarotstrahler kann dabei zum Erzielen der Differenz in der an den Benutzer in dem Hautbereich abgegebenen Wärmeleistung infolge eines veränderlichen Heizstroms ausgebildet sein. Allgemein kann die Wärmebehandlungsvorrichtung dazu ausgebildet sein, das Verändern der an den Benutzer abgegebenen Wärmeleistung durch entsprechendes Verändern einer mittels des Infrarotstrahlers erzeugten Strahlungsintensität zu erzielen.

Die Steuerungseinrichtung und der Infrarotstrahler können dazu ausgebildet sein, die Differenz in der an den Benutzer in dem Hautbereich abgegebenen Wärmeleistung wenigstens teilweise durch anteiliges Verändern des Heizstroms, d.h. nicht beschränkt auf ein binäres Ein- bzw. Ausschalten eines identischen Heizstroms, zu erzielen.

Die Wärmequelle kann ein keramikfreies wärmeerzeugendes Element umfassen. Zudem kann die Wärmequelle eine steuerbare Halogenlampe, eine steuerbare Infrarot-, IR-, Leuchtdiode, LED, und/oder ein steuerbares Kohlefaser-Heizelement umfassen. Insbesondere kann die Wärmequelle eine steuerbare Halogenlampe umfassen, die als wärmeerzeugendes Element wenigstens einen Glühdraht umfasst. Zusätzlich oder alternativ kann die Wärmequelle eine steuerbare IR-LED umfassen, die als wärmeerzeugendes Element wenigstens eine Halbleiteranordnung umfasst. Zusätzlich oder alternativ kann die Wärmequelle ein steuerbares Kohlefaser-Heizelement umfassen, das als wärmeerzeugendes Element wenigstens eine mit einem Heizstrom beaufschlagbare Kohlefaser umfasst.

Die Wärmequelle kann eine absorptionsfilterlose Halogenlampe umfassen. Dadurch ist vermeidbar, dass ein Filter des Infrarotstrahlers, beispielsweise ein keramik- oder metallbeschichtetes Deckglas, infolge von (Teil-)Absorption der mittels des Infrarotstrahlers erzeugten Infrarotstrahlung erwärmt wird und durch das Ansammeln von Wärme bzw. durch Abstrahlen von angesammelter Wärme ein Verändern der an den Benutzer mittels des Infrarotstrahlers abgegebenen Wärmeleistung verzögert.

Das Verwenden einer Wärmequelle, die eine Halogenlampe umfasst, ist bei vielen Anwendungen vorteilhaft wegen eines verhältnismäßig breiten Wellenlängenspektrums der Infrarotstrahlung, die mittels einer Halogenlampe erzeugbar ist. Demgegenüber erzeugen IR-LEDs Infrarotstrahlung funktionsbedingt in schmalen Wellenlängenbändern.

Das Verwenden einer Wärmequelle, die eine Halogenlampe umfasst, ist bei einigen der vorliegend beschriebenen Anwendungen zudem vorteilhaft wegen einer gegenüber Kohlefaser-Heizelementen typischerweise geringeren Verzögerung eines Veränderns der an den Benutzer mittels des Infrarotstrahlers abgebbaren Wärmeleistung.

Der Infrarotstrahler kann in dem Betriebszustand, der einer an den Benutzer abgegebenen Wärmeleistung im Bereich von 20 bis 120 Milliwatt pro Quadratzentimeter in dem Hautbereich des Benutzers entspricht, eine minimale Verzögerung von weniger als 4, vorzugsweise weniger als 3, bevorzugt weniger als 2,5 Sekunden zum Erzielen einer Differenz von wenigstens 30%, vorzugsweise wenigstens 35%, bevorzugt wenigstens 40% in der an den Benutzer in dem Hautbereich abgegebenen Wärmeleistung aufweisen.

Zusätzlich oder alternativ kann der Infrarotstrahler in dem Betriebszustand, der einer an den Benutzer abgegebenen Wärmeleistung im Bereich von 20 bis 120 Milliwatt pro Quadratzentimeter in dem Hautbereich des Benutzers entspricht, eine minimale Verzögerung von wenigstens 0,5, insbesondere wenigstens 1 Sekunde zum Erzielen einer Differenz von wenigstens 50%, insbesondere wenigstens 80% in der an den Benutzer in dem Hautbereich abgegebenen Wärmeleistung aufweisen.

Die Steuerungseinrichtung kann dazu ausgebildet sein, die Heizleistung wenigstens teilweise im Rahmen einer Heizleistungsregelung auf der Grundlage der erfassten Oberflächentemperatur zu steuern.

Die Steuerungseinrichtung kann dabei dazu ausgebildet sein, die Wärmebehandlungsvorrichtung zum Bestimmen eines benutzerspezifischen Wärmeabbauvermögens in dem Hautbereich des Benutzers zu steuern. Ferner kann die Steuerungseinrichtung dazu ausgebildet sein, nach einem Bestimmen des benutzerspezifischen Wärmeabbauvermögens die Heizleistungsregelung gemäß einer Regelspanne, die wenigstens teilweise durch das benutzerspezifische Wärmeabbauvermögen bestimmt ist, durchzuführen. Eine Effizienz der Heizleistungsregelung lässt sich so verbessern.

Zusätzlich oder alternativ kann die Steuerungseinrichtung dazu ausgebildet sein, nach einem Bestimmen des benutzerspezifischen Wärmeabbauvermögens die Heizleistungsregelung gemäß einer erwarteten Körperreaktion des Benutzers, die wenigstens teilweise durch das benutzerspezifische Wärmeabbauvermögen bestimmt ist, durchzuführen. Eine genauere und sicherere Steuerung der Heizleistung des Infrarotstrahlers ist so ermöglicht.

Das Bestimmen des benutzerspezifischen Wärmeabbauvermögens kann ein Bestimmen einer Ausgangsoberflächentemperatur in dem Hautbereich des Benutzers umfassen. Ferner kann das Bestimmen ein Verändern einer Heizleistung des Infrarotstrahlers derart, dass in einem Zeitraum von weniger als 5 Sekunden eine Differenz von wenigstens 20% in der an den Benutzer abgegebenen Wärmeleistung in dem Hautbereich des Benutzers bewirkt ist, und ein Bestimmen eines Temperaturverlaufs in dem Hautbereich des Benutzers wenigstens teilweise auf der Grundlage einer Oberflächentemperatur in dem Hautbereich des Benutzers, die mittels des Wärmesensors nach dem Verändern der Heizleistung erfasst ist, umfassen. Der Temperaturverlauf weist dabei auf ein Wärmeabbauvermögen des Benutzers hin.

Das Bestimmen der Ausgangsoberflächentemperatur kann ein Erwärmen des Hautbereichs des Benutzers mittels Infrarotstrahlung, die mittels des Infrarotstrahlers erzeugt ist, zum Erreichen der Ausgangsoberflächentemperatur umfassen. Dabei kann das Verändern der Heizleistung ein Verringern der Heizleistung umfassen. Der Temperaturverlauf entspricht dabei einem Abkühlen des Hautbereichs wenigstens teilweise infolge von Wärmeabbau im Körper des Benutzers.

Alternativ kann das Verändern der Heizleistung ein Erhöhen der Heizleistung umfassen. Der Temperaturverlauf entspricht dabei einem Aufwärmen des Hautbereichs, das wenigstens teilweise durch Wärmeabbau im Körper des Benutzers bestimmt, insbesondere verzögert, ist.

Die Heizleistungsregelung kann eine maximale obere Schwellenwerttemperatur von weniger als 45 Grad Celsius, vorzugsweise weniger als 44,5 Grad Celsius, bevorzugt nicht mehr als 44 Grad Celsius aufweisen. Dadurch kann gewährleistet sein, dass im Rahmen üblicher Dauern von Wärmebehandlungen, beispielsweise bis zu Höchstdauern von wenigstens 2 Stunden, vorzugsweise wenigstens 3 Stunden, bevorzugt wenigstens 4 Stunden, Hautschäden infolge einer Überhitzung vermieden werden.

Die Steuerungseinrichtung kann eine Speichervorrichtung umfassen. Die Speichervorrichtung kann dazu ausgebildet sein, Programmcode zum Betreiben der Wärmebehandlungsvorrichtung gemäß wenigstens einem Wärmebehandlungsprogramm zu speichern.

Die Speichervorrichtung kann Programmcode zum Betreiben der Wärmebehandlungsvorrichtung gemäß wenigstens einem Wärmebehandlungsprogramm enthalten, das während einer Wärmebehandlung in wenigstens einem Zeitabschnitt von weniger als 5 Sekunden eine Differenz von wenigstens 20% in der an den Benutzer abgegebenen Wärmeleistung in dem Hautbereich des Benutzers vorsieht. Das Wärmebehandlungsprogramm kann ein Programm für eine Wärmereizbehandlung umfassen.

Die Wärmebehandlungsvorrichtung kann ferner wenigstens ein Spiegelelement, das dazu ausgebildet ist, Infrarotstrahlung, die mittels des Infrarotstrahlers ausgesendet ist, in Richtung auf den Hautbereich des Benutzers umzulenken, umfassen. Dabei kann der Wärmesensor in einem Bereich des Spiegelelements angeordnet sein. Ein Erfassungsbereich des Wärmesensors kann sich wenigstens teilweise in einem Abschnitt eines Strahlungskegels, der durch die umgelenkte Infrarotstrahlung bestimmt ist, erstrecken.

Die Wärmebehandlungsvorrichtung kann wenigstens zwei Spiegelelemente und wenigstens zwei Infrarotstrahler, die an verschiedenen Seiten in Bezug auf die Spiegelelemente angeordnet sind, umfassen. Die wenigstens zwei Spiegelelemente können als einstückiges Element ausgebildet sein.

Die Wärmequelle jedes der Infrarotstrahler kann eine steuerbare Halogenröhre umfassen. Die Halogenröhren können zumindest im Wesentlichen parallel zueinander ausgerichtet sein. Eine Spiegelfläche jedes der Spiegelelemente kann zumindest im Wesentlichen parallel zu einer Längserstreckungsrichtung wenigstens einer der Halogenröhren ausgerichtet sein.

Die Wärmebehandlungsvorrichtung kann eine Mehrzahl von Wärmesensoren umfassen, die dazu angeordnet sind, eine Oberflächentemperatur in jedem von verschiedenen Teilbereichen des Hautbereichs des Benutzers zu erfassen. Dabei kann die Steuerungseinrichtung dazu ausgebildet sein, die Heizleistung wenigstens teilweise auf der Grundlage der erfassten Oberflächentemperatur in den verschiedenen Teilbereichen des Hautbereichs des Benutzers zu steuern.

Der Hautbereich des Benutzers kann eine Fläche von wenigstens 400 Quadratzentimetern aufweisen.

Die Wärmebehandlungsvorrichtung kann derart ausgebildet sein, dass in einem Volumen, das sich ausgehend von dem Hautbereich und bis zu einem Abstand von wenigstens 10 Zentimetern von wenigstens einer Stelle des Hautbereichs parallel zu einer Einfallsrichtung der Infrarotstrahlung erstreckt, eine ortsabhängige Leistungsdichte der Infrarotstrahlung an jedweden verschiedenen Stellen des Volumens um höchstens 8% verschieden ist.

Gemäß einem weiteren Aspekt wird eine Wärmekabine vorgestellt, genauer gesagt eine Infrarotkabine. Die Wärmekabine umfasst eine Wärmebehandlungsvorrichtung der hier vorgestellten Art.

Die Wärmekabine kann als Druckkammer, insbesondere zum Zweck hyperbarer Therapieanwendungen, ausgebildet sein. Die Wärmebehandlungsvorrichtung kann dabei zum Einsatz während einer hyperbaren Therapieanwendung ausgebildet sein.

Die vorstehend beschriebene Wärmekabine für hyperbare Therapieanwendungen zeigt, dass die offenbarte Wärmebehandlungsvorrichtung für den Einsatz in verschiedenen Umgebungen und Situationen geeignet ist und eingesetzt werden kann.

Gemäß einem weiteren Aspekt wird ein Verfahren zum benutzerbezogenen Kalibrieren einer Wärmebehandlungsvorrichtung vorgestellt. Bei der Wärmebehandlungsvorrichtung handelt es sich um eine Wärmebehandlungsvorrichtung der hier vorgestellten Art. Das Verfahren umfasst Bestimmen einer Ausgangsoberflächentemperatur in dem Hautbereich des Benutzers. Das Verfahren umfasst ferner Verändern, mittels der Steuerungseinrichtung und des Infrarotstrahlers, einer Heizleistung des Infrarotstrahlers derart, dass in einem Zeitraum von weniger als 5 Sekunden eine Differenz von wenigstens 20% in der an den Benutzer abgegebenen Wärmeleistung in dem Hautbereich des Benutzers bewirkt ist, Erfassen, mittels des Wärmesensors und nach dem Verändern der Heizleistung, einer Oberflächentemperatur in dem Hautbereich des Benutzers, und Bestimmen, mittels der Steuerungseinrichtung und wenigstens teilweise auf der Grundlage der erfassten Oberflächentemperatur, eines Temperaturverlaufs in dem Hautbereich des Benutzers. Der Temperaturverlauf weist auf ein Wärmeabbauvermögen des Benutzers hin. Das Verfahren umfasst außerdem Bestimmen, mittels der Steuerungseinrichtung und wenigstens teilweise auf der Grundlage des Wärmeabbauvermögens des Benutzers, einer Regelspanne für eine Heizleistungsregelung der Wärmebehandlungsvorrichtung und/oder Speichern, mittels einer Speichervorrichtung der Steuerungseinrichtung, wenigstens eines benutzerbezogenen Kennwerts, der auf das Wärmeabbauvermögen des Benutzers hinweist.

### Kurze Beschreibung der Zeichnungen

Weitere Merkmale, Vorzüge und Ziele der Erfindung werden anhand der Zeichnungen und der ausführlichen Beschreibung deutlich. Es zeigen:
Fig. 1 eine Wärmebehandlungsvorrichtung gemäß einem Beispiel;
Fig. 2 eine Wärmekabine gemäß einem Beispiel, und
Fig. 3 ein Verfahren zum Kalibrieren einer Wärmebehandlungsvorrichtung gemäß einem Beispiel.

### Ausführliche Beschreibung

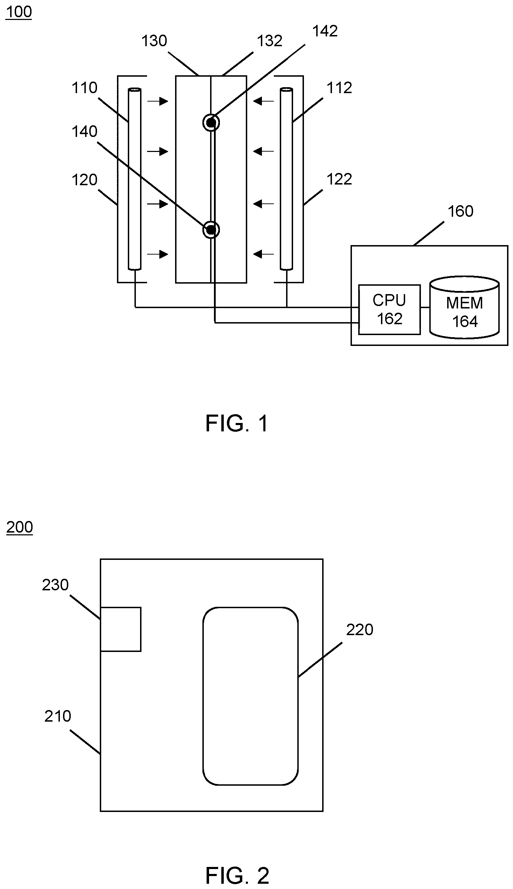

Fig. 1 zeigt schematisch und exemplarisch eine Wärmebehandlungsvorrichtung 100. Die Wärmebehandlungsvorrichtung 100 umfasst mehrere Infrarotstrahler 110, 112. Jeder der Infrarotstrahler 110, 112 ist jeweils in einem Gehäusebereich 120, 122 der Wärmebehandlungsvorrichtung 100 angeordnet. Die Wärmebehandlungsvorrichtung 100 umfasst außerdem mehrere Spiegelelemente 130, 132 sowie mehrere Wärmesensoren 140, 142, die im Bereich der Spiegelelemente 130, 132 angeordnet sind. Die Wärmebehandlungsvorrichtung 100 umfasst außerdem eine Steuerungseinrichtung 160 mit einer Prozessoreinheit 162 und einer Speichervorrichtung 164. Die Steuerungseinrichtung 160 ist mit den Wärmesensoren 140, 142 und mit den Infrarotstrahlern 110, 112 operativ verbunden.

Die Infrarotstrahler 110, 112 sind auf verschiedenen Seiten der Spiegelelemente 130, 132 angeordnet. Wie in Fig. 1 durch Pfeile schematisch dargestellt, ist jeder der Infrarotstrahler 110, 112 so angeordnet, dass Infrarotstrahlung, die mittels einer Wärmequelle jedes der Infrarotstrahler 110, 112 erzeugt wird, in Richtung auf die Spiegelelemente 130, 132 abgestrahlt wird.

Die Spiegelelemente 130, 132 sind so angeordnet, dass Infrarotstrahlung, die von jedwedem der Infrarotstrahler 110, 112 abgestrahlt wird, auf eine Spiegelfläche jeweils wenigstens eines der Spiegelelemente 130, 132, das dem jeweiligen Infrarotstrahler 110, 112 zugewandt angeordnet ist, trifft. Die Spiegelelemente 130, 132 sind so ausgerichtet, dass Infrarotstrahlung, die von einem jeweils zugeordneten Infrarotstrahler 110, 112 kommend auf wenigstens eines der Spiegelelemente 130, 132 trifft, in Richtung auf einen Benutzer der Wärmebehandlungsvorrichtung 100 umgelenkt wird. Der Benutzer befindet sich dabei in einem Bestrahlungsbereich der Wärmebehandlungsvorrichtung 100. In dem Beispiel von Fig. 1 befindet sich der Bestrahlungsbereich der Wärmebehandlungsvorrichtung 100 oberhalb der Zeichnungsebene. Dabei sind die Spiegelelemente 130, 132 beispielsweise dachförmig bzw. in Form eines umgedrehten ,V' in Bezug auf die Zeichnungsebene angeordnet. Infrarotstrahlung, die von den Infrarotstrahlern 130, 132 seitlich auf jeweils eines der Spiegelelemente 130, 132 trifft, wird so mittels der Spiegelelemente 130, 132 in eine Richtung senkrecht zur Zeichnungsebene umgelenkt.

Die Wärmesensoren 140, 142 sind zwischen den Spiegelelementen 130, 132 angeordnet. Die Spiegelelemente 130, 132 weisen dazu beispielsweise Aussparungen auf. Dies gestattet eine Anordnung der Wärmesensoren 140, 142 hinter den Spiegelelementen 130, 132. Auf diese Weise sind die Wärmesensoren 140, 142 vor direkter Infrarotstrahlung, die von den Infrarotstrahler 110, 112 abgestrahlt wird und die sonst ein Messergebnis der Wärmesensoren 140, 142 verfälschen könnte, abgeschirmt. Die Wärmesensoren 140, 142 sind so ausgerichtet, dass ein Erfassungsbereich jedes der Wärmesensoren 140, 142 mit dem Bestrahlungsbereich der Wärmebehandlungsvorrichtung 100 wenigstens teilweise überlappt.

Die in Fig. 1 gezeigte Anordnung der Infrarotstrahler 110, 112, der Spiegelelemente 130, 132 und der Wärmesensoren 140, 142 gestattet das Aufbringen von Wärme in Form von Wärmestrahlung auf einen Benutzer bzw. einen Hautbereich des Benutzers in dem Bestrahlungsbereich der Wärmebehandlungsvorrichtung 100. Gleichzeitig ist ermöglicht, mittels der Wärmesensoren 140, 142 eine Oberflächentemperatur des Hautbereichs während des Aufbringens von Wärmestrahlung mittels der Wärmebehandlungsvorrichtung 100 in dem Hautbereich zu erfassen. Eine Wirkung der Wärmestrahlung auf die Oberflächentemperatur in dem bestrahlten Hautbereich lässt sich so, beispielsweise kontinuierlich oder in beliebigen zeitlichen Abständen, sensorisch überwachen.

Die Steuerungseinrichtung 160 ist dazu ausgebildet, eine Heizleistung jedes der Infrarotstrahler 110, 112 zu steuern. Dazu ist die Steuerungseinrichtung 160 mit den Infrarotstrahlern 110, 112 operativ verbunden. Die Verbindung erfolgt beispielsweise über einen Dimmer (nicht dargestellt), der mittels der Steuerungseinrichtung 160 steuerbar ist. In anderen Beispielen ist die Steuerungseinrichtung 160 dazu ausgebildet, an jeden der Infrarotstrahler 110, 112 einen gemäß einer vorgesehenen Heizleistung bemessenen Heizstrom auszugeben.

Die Steuerungseinrichtung 160 ist ferner dazu ausgebildet, von jedem der Wärmesensoren 140, 142 Sensorsignale zu empfangen, die auf eine mittels des jeweiligen Sensors 140, 142 erfasste Oberflächentemperatur hinweisen. Ein Sensorsignal von jedwedem der Wärmesensoren 140, 142 weist dabei auf eine Temperatur in dem Erfassungsbereich des jeweiligen Wärmesensors 140, 142 in.

Die Steuerungseinrichtung 160 ist dazu ausgebildet, eine Heizleistung jedes der Infrarotstrahler 110, 112 wenigstens teilweise auf der Grundlage einer mittels der Wärmesensoren 140, 142 erfassten Oberflächentemperatur in einem Hautbereich des Benutzers, der sich im Bestrahlungsbereich der Wärmebehandlungsvorrichtung 100 befindet, zu steuern, insbesondere temperaturabhängig zu regeln. Dafür ist die Prozessoreinheit 162 dazu programmiert, ein oder mehrere Sensorsignale, die mittels eines oder mehrerer der Wärmesensoren 140, 142 erzeugt und an die Steuerungseinrichtung 160 ausgegeben und von der Steuerungseinrichtung 160 empfangen werden, mit wenigstens einer Bezugstemperatur zu vergleichen und in Abhängigkeit von dem Vergleichsergebnis die Heizleistung wenigstens eines der Infrarotstrahler 110, 112 zu verändern. Ein Verändern der Heizleistung kann dabei einem Verringern oder einem Erhöhen der Heizleistung entsprechen.

In einigen Beispielen der Wärmebehandlungsvorrichtung 100 ist eine Funktionalität der Prozessoreinheit 162 durch Programmcode bestimmt, der mittels der Speichervorrichtung 164 gespeichert ist und von der Prozessoreinheit 162 ausgeführt wird. In einigen Beispielen erfolgt zudem eine Heizleistungsregelung mittels der Steuerungseinrichtung 160 durch Vergleichen einer erfassten Temperatur mit einer oder mehreren Bezugstemperaturen, die mittels der Speichervorrichtung 164 gespeichert sind.

Die Infrarotstrahler 110, 112 sind derart steuerbar, dass sie in einem Betriebszustand, der einer an den Benutzer der Wärmebehandlungsvorrichtung 100 abgegebenen Wärmeleistung im Bereich von 20 bis 120 Milliwatt pro Quadratzentimeter in dem Hautbereich des Benutzers entspricht, eine minimale Verzögerung von weniger als 5 Sekunden zum Erzielen einer Differenz von wenigstens 20% in der an den Benutzer in dem Hautbereich abgegebenen Wärmeleistung aufweisen. Dabei umfasst in einigen Beispielen eine Wärmequelle in jedem der Infrarotstrahler 110, 112 ein keramikfreies wärmeerzeugendes Element. Eine keramikfreie, oder in anderen Beispielen andersartige, Wärmequelle in jedwedem der Infrarotstrahler 110, 112 umfasst zudem beispielsweise eine steuerbare Halogenlampe, beispielsweise eine steuerbare Halogenröhre, eine steuerbare Infrarot-, IR-, Leuchtdiode, LED, und/oder ein steuerbares Kohlefaser-Heizelement.

Die Verwendung von Infrarotstrahlen 110, 112 wie voranstehend beschriebenen ermöglicht verhältnismäßig kurzfristige Änderungen der an den Benutzer der Wärmebehandlungsvorrichtung 100 abgegebenen Wärmeleistung. Auf diese Weise sind verhältnismäßig kurze Regelspannen zum Regeln der Heizleistung der Infrarotstrahler 110, 112 mittels der Steuerungseinrichtung 160 wirksam verwendbar. Eine Oberflächentemperatur in dem bestrahlten Hautbereich eines Benutzers ist so genauer einstellbar, da ein Über- oder Untersteuern bei der Regelung der Heizleistung verringert werden kann. So ist eine Verletzungsgefahr bzw. die Wahrscheinlichkeit einer weniger wirksamen Wärmebehandlung als jeweilige Folgen eines Über- bzw. Untersteuerns verringert.

Die Verwendung von Infrarotstrahlern 110, 112 wie voranstehend beschrieben gestattet gleichzeitig erweiterte Anwendungen der Wärmebehandlungsvorrichtung 100. Dazu zählt das Bestimmen eines Wärmeabbauvermögens eines jeweiligen Benutzers der Wärmebehandlungsvorrichtung 100. Das Wärmeabbauvermögen ist bestimmt durch das Vermögen des Körpers, Wärme im Bereich der Haut in tieferliegende Regionen des Körpers abzuführen. Das Wärmeabbauvermögen beeinflusst daher einen Temperaturverlauf in dem bestrahlten Hautbereich während einer Wärmebehandlung in Bezug auf eine Wärmeleistung, die mittels der Wärmebehandlungsvorrichtung 100 an den Benutzer abgegeben wird, und eine Bestrahlungsdauer. Eine Kenntnis von dem benutzerspezifischen Wärmeabbauvermögen gestattet daher eine verbesserte Steuerung der Heizleistung der Infrarotstrahler 110, 112 mittels der Steuerungseinrichtung 160 gemäß einer vorgesehenen Temperatur. Beispielsweise kann so eine Wirkung einer Heizleistungsänderung auf die sich dadurch ergebende Änderung der Oberflächentemperatur in dem bestrahlten Hautbereich des Benutzers im Voraus benutzerspezifisch eingeschätzt werden. Eine Kenntnis von dem Wärmeabbauvermögen eines Benutzers ist außerdem verwendbar, um eine Regelspanne der Steuerungseinrichtung 160 benutzerspezifisch gemäß einer zeitlichen Rate zu bestimmen, in der der Benutzer aufgebrachte Wärme abbaut. Eine Effizienz der Regelung lässt sich so verbessern.

Um das Wärmeabbauvermögen eines Benutzers zu bestimmen ist die Steuerungseinrichtung 160 in einigen Beispielen der Wärmebehandlungsvorrichtung 100 dazu programmiert, den Hautbereich des Benutzers bis zum Erreichen einer bestimmten Ausgangsoberflächentemperatur und/oder über einen bestimmten Zeitraum, um eine geeignete Ausgangsoberflächentemperatur zu erreichen, zu erwärmen. Die Wärmebehandlungsvorrichtung 100 ist ferner dazu programmiert, die Heizleistung der Infrarotstrahler 110, 112 anschließend zu verringern, sodass kein weiteres Aufwärmen, sondern ein Abkühlen des Hautbereichs erfolgt. Die Heizleistung der Infrarotstrahler 110, 112 wird dabei derart verringert, dass in einem Zeitraum von weniger als 5 Sekunden eine Differenz von wenigstens 20% in der an den Benutzer abgegebenen Wärmeleistung in dem Hautbereich bewirkt ist. Zum Bestimmen des Wärmeabbauvermögens des Benutzers wird anschließend, d.h. nach dem Verringern der Heizleistung, eine Oberflächentemperatur in dem Hautbereich erfasst und anhand der erfassten Temperatur ein Temperaturverlauf in dem Hautbereich quantifiziert. Der Temperaturverlauf ist zumindest teilweise durch das Wärmeabbauvermögen des Benutzers bestimmt. Beispielsweise kann durch Vergleichen des Temperaturverlaufs mit gespeicherten Bezugsverlaufsdaten mittels der Steuerungseinrichtung 160 ein, beispielsweise relatives, Wärmeabbauvermögen des Benutzers ermittelt werden. Ein Kennwert für das derart bestimmte Wärmeabbauvermögen des Benutzers wird in einigen Beispielen zur späteren Verwendung in der Speichervorrichtung 164 gespeichert.

In weiteren Beispielen der Wärmebehandlungsvorrichtung 100 ist die Steuerungseinrichtung 160 dazu programmiert, um das Wärmeabbauvermögen eines Benutzers zu bestimmen, ausgehend von einer bestimmten Ausgangsoberflächentemperatur in dem Hautbereich des Benutzers, die beispielsweise einer ausgeglichenen Hauttemperatur bei gegebener Raumtemperatur entspricht, die Heizleistung der Infrarotstrahler 110, 112 zu erhöhen, sodass ein Aufwärmen des Hautbereichs einsetzt. Die Heizleistung der Infrarotstrahler 110, 112 wird dabei derart erhöht, dass in einem Zeitraum von weniger als 5 Sekunden eine Differenz von wenigstens 20% in der an den Benutzer abgegebenen Wärmeleistung in dem Hautbereich bewirkt ist. Zum Bestimmen des Wärmeabbauvermögens des Benutzers wird anschließend, d.h. nach dem Erhöhen der Heizleistung und während des anhaltenden Abgebens von Wärmeleistung an den Benutzer, eine Oberflächentemperatur in dem Hautbereich erfasst und anhand der erfassten Temperatur ein Temperaturverlauf in dem Hautbereich quantifiziert. Der Temperaturverlauf ist dabei insbesondere durch einen Temperaturanstieg gekennzeichnet, der zumindest teilweise durch das Wärmeabbauvermögen des Benutzers bestimmt, insbesondere verzögert, ist. Durch Vergleichen des Temperaturverlaufs mit gespeicherten Bezugsverlaufsdaten mittels der Steuerungseinrichtung 160 lässt sich so wiederum ein, beispielsweise relatives, Wärmeabbauvermögen des Benutzers ermitteln und ein Kennwert für das derart bestimmte Wärmeabbauvermögen des Benutzers zur späteren Verwendung in der Speichervorrichtung 164 speichern.

Das beschriebene Bestimmen des Wärmeabbauvermögens ist begünstigt durch die Infrarotstrahler 110, 112, die ein verhältnismäßig kurzfristiges Verändern der an den Benutzer abgegebenen Wärmeleistung gestatten. Auf diese Weise ist ein Zeitraum, über den sich das Verändern der Wärmeleistung erstreckt, kurz, und dadurch ein Zeitpunkt, ab dem der Temperaturverlauf vorteilhaft zu quantifizieren ist, mit hoher Genauigkeit bestimmbar. Dies gestattet eine verhältnismäßig genaue Berechnung des benutzerspezifischen Wärmeabbauvermögens auf effiziente Weise.

Die Verwendung der Infrarotstrahler 110, 112 wie voranstehend beschrieben gestattet zudem eine Erweiterung von Wärmebehandlungen, die mittels der Wärmebehandlungsvorrichtung 100 durchführbar sind. Sie gestattet insbesondere die Durchführung von Wärmebehandlungen, die im zeitlichen Verlauf der betreffenden Wärmebehandlung in wenigstens einem Zeitabschnitt von weniger als 5 Sekunden eine Differenz von wenigstens 20% in der den Benutzer abgegebenen Wärmeleistung vorsehen. Das ermöglicht beispielsweise die Durchführung von Wärmereizbehandlungen, die kurzfristiges Verändern, d.h. kurzfristiges Verringern und/oder kurzfristiges Erhöhen, der an den Benutzer in dem Hautbereich abgegebenen Wärmeleistung erfordern.

Das Verändern der an den Benutzer in dem Hautbereich abgegebenen Wärmeleistung erfolgt dabei beispielsweise mit einer Verzögerung, die geringer ist, als es zum Einstellen eines thermodynamischen Gleichgewichts zwischen einer an den Benutzer währenddessen zu einem jeweiligen Zeitpunkt abgegebenen Wärmeleistung und einer sich infolge des Veränderns der Wärmeleistung gleichzeitig verändernden Oberflächentemperatur in dem Hautbereich erforderlich ist.

Wärmereizbehandlungen der beschriebenen Art lassen sich dazu verwenden, eine Adrenalinausschüttung im Körper des Benutzers hervorzurufen bzw. zu steigern. Dies ist beispielsweise für sogenannte Aktivierungsanwendungen vorteilhaft, die eine belebende und aufmunternde Wirkung haben.

Zusätzlich oder alternativ lassen sich Wärmereize der beschriebenen Art, etwa im Verlauf einer Wärmebehandlung, dazu nutzen, durch die jeweils hervorgerufene bzw. gesteigerte Adrenalinausschüttung eine zeitweilige Verengung der Blutgefäße herbeizuführen, um einen Wärmeabbau in dem Hautbereich zu beeinträchtigen.

Das ist beispielsweise nutzbar, um einen effektiven Wärmeeintrag in einem Gewebebereich zu erhöhen. So lässt sich beispielsweise ein sogenanntes Wärmedepot in dem betreffenden Gewebebereich erzeugen. Wärmedepots sind geeignet, auf effiziente Weise dem Benutzer ein anhaltendes Wärmegefühl über einen längeren Zeitraum nach der Wärmebehandlung zu vermitteln. Ein erhöhter effektiver Wärmeeintrag, wie er sich durch Wärmereize der beschriebenen Art im Verlauf einer Wärmebehandlung erzielen lässt, ist zudem für Muskelentspannungsanwendungen vorteilhaft.

Mittels der beschriebenen Merkmale gestattet die Wärmebehandlungsvorrichtung 100 zudem eine zuverlässige Regelung der Heizleistung auf der Grundlage eines vorgegebenen Anstiegs der Hauttemperatur auch bei vorgegebenen "steilen" Anstiegen, d.h. bei vorgegebenen Erhöhungen der Hauttemperatur in verhältnismäßig kurzen Zeiträumen.

Während eine einfache, schonende Temperaturerhöhung im Gewebe Wärmezufuhr über längere Zeit vorsieht, beruht die Nutzung von Hautreflexzonen zur Implementierung physiologischer Effekte auf schnellen Temperaturveränderungen der Haut. Die Wärmebehandlungsvorrichtung 100 begünstigt auf die beschriebene Weise verschiedene Anwendungen. Schnelle Temperaturveränderungen erfordern dazu ein schnell reagierendes Infrarotstrahlung bereitstellendes System. Kontrolle durch ein geeignetes Sicherungssystem, das beispielsweise durch geeignete Regelung umsetzbar ist, gestattet dabei gleichzeitig das Verhindern akzidentieller thermischer Schädigungen der Haut.

In einigen Beispielen der Wärmebehandlungsvorrichtung 100 sind entsprechende Programmvorschriften zur Durchführung einer oder mehrerer Wärmebehandlungen der voranstehend beschriebenen Art in der Speichervorrichtung 164 gespeichert.

Die Verwendung der Infrarotstrahler 110, 112 wie voranstehend beschrieben gestattet zudem eine verbesserte Homogenität einer Wärmeleistungsverteilung in dem Bestrahlungsbereich der Wärmebehandlungsvorrichtung 100. Gegenüber Keramikwärmestrahlern umfassen die Infrarotstrahler 110, 112 als Wärmequelle beispielsweise Halogenlampen, IR-LEDs, oder Kohlefaserelemente, deren wärmeerzeugendes Element eine verhältnismäßig kleine räumliche Erstreckung hat. Das begünstigt eine Strahlungsführung zwischen dem jeweiligen wärmeerzeugenden Element und dem Bestrahlungsbereich, beispielsweise mittels Reflektoren. Bei der Wärmebehandlungsvorrichtung 100 wie in Fig. 1 gezeigt erfolgt eine Strahlungsführung beispielsweise mittels der Spiegelelemente 130, 132. Ferner umfassen in einigen Beispielen die Infrarotstrahler 110, 112 Reflektoren, die erzeugte Infrarotstrahlung verstärkt in Richtung auf die Spiegelelemente 130, 132 lenken. Zudem ist in einigen Beispielen der Wärmebehandlungsvorrichtung 100 auch in den Gehäusebereichen 120, 122 jeweils eine Anordnung eines oder mehrerer strahlungsführender Elemente vorgesehen.

Wie in Fig. 1 schematisch dargestellt, sind in einigen Beispielen der Wärmebehandlungsvorrichtung 100 die Infrarotstrahler 110, 112 als steuerbare Halogenröhren ausgebildet, die im Wesentlichen parallel zueinander sowie parallel zu den Spiegelelementen 130, 132 angeordnet sind. Die gezeigte Anordnung ist vorteilhaft bei einer Verwendung der Wärmebehandlungsvorrichtung 100 zum Aufbringen von Wärme auf einen langgestreckten Hautbereich, beispielsweise entlang der Wirbelsäule, wobei eine möglichst gleichmäßige Verteilung der Wärmeleistung über den gesamten Hautbereich gewünscht ist.

Die Wärmebehandlungsvorrichtung 100 wie in Fig. 1 gezeigt begünstigt außerdem eine verhältnismäßig gleichmäßige Leistungsdichte der Infrarotstrahlung in einem Volumen oberhalb des Hautbereichs, beispielsweise einem Volumen, das sich angrenzend an den Hautbereich von einer tiefsten Stelle in Strahlungsrichtung gemessen um etwa 10 cm entgegen der Strahlungsrichtung erstreckt. Selbst bei Unebenheiten des bestrahlten Hautbereichs, wie sie beispielsweise durch die Wirbelsäulenkrümmungen auftreten, ist so ein gleichmäßiger Wärmeeintrag in dem gesamten Hautbereich möglich. Dabei wirkt sich die Möglichkeit zur verbesserten Strahlungsführung, wie sie durch die Infrarotstrahler 110, 112 ermöglicht ist, begünstigend aus.

Fig. 2 zeigt schematisch und exemplarisch eine Wärmekabine 200. Die Wärmekabine 200 umfasst eine Kabinenwand 210, die einen Innenraum der Wärmekabine 200 umgibt. Die Wärmekabine 200 umfasst außerdem eine Wärmebehandlungsvorrichtung 220 und eine Überdruckvorrichtung 230.

Bei der Wärmebehandlungsvorrichtung 220 handelt es sich um eine Wärmebehandlungsvorrichtung wie voranstehend im Zusammenhang mit der Wärmebehandlungsvorrichtung 100 in Fig. 1 beschrieben. Zur Benutzung der Wärmebehandlungsvorrichtung 220 betritt ein Benutzer den Innenraum der Wärmekabine 210. Gegenüber einer freistehenden Wärmebehandlungsvorrichtung begünstigt die Wärmekabine 210 beispielsweise eine Durchwärmung des gesamten Körpers des Benutzers.

Die Überdruckvorrichtung 230 ist dazu vorgesehen, einen Überdruck in dem Innenraum der Wärmekabine 200 zu erzeugen. Die Wärmekabine 210 ist dabei als Druckkammer zum Zweck hyperbarer Therapieanwendungen ausgebildet. Die Wärmekabine 200 ist dabei ferner dazu vorgesehen, Wärmebehandlungen mittels der Wärmebehandlungsvorrichtung 220 gleichzeitig mit hyperbaren Therapieanwendungen zu ermöglichen.

Eine Überdruckumgebung wirkt sich auf den Betrieb der Wärmebehandlungsvorrichtung 220 dahingehend aus, dass bei einem erhöhten Luftdruck die Strahlungsabsorption durch die Luft zwischen den Infrarotstrahlern 110, 112 und dem zu bestrahlenden Hautbereich signifikant erhöht ist. Die Infrarotstrahler 110, 112 wie voranstehend beschrieben begünstigen dabei die Bereitstellung von Infrarotstrahlung auch in einem entsprechend höheren Heizleistungsbereich sowie eine dynamische Anpassung der Heizleistung auch an sich ändernde Druckverhältnisse im Innenraum der Wärmekabine 210.

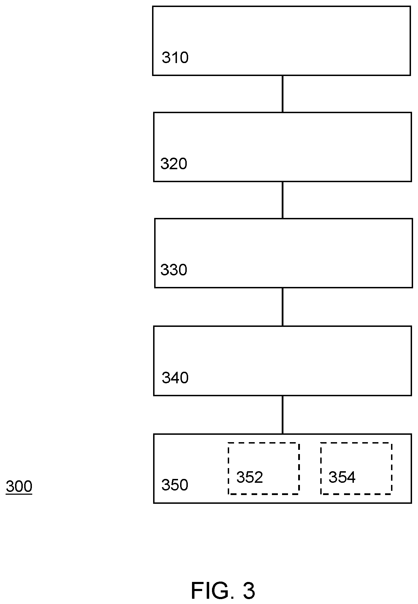

Fig. 3 zeigt ein Flussdiagramm eines Verfahrens 300 zum Kalibrieren einer Wärmebehandlungsvorrichtung gemäß einem benutzerspezifischen Wärmeabbauvermögen. Bei der Wärmebehandlungsvorrichtung handelt es sich um eine Wärmebehandlungsvorrichtung der voranstehend im Zusammenhang mit Fig. 1 und 2 beschriebenen Art.

Das Verfahren 300 umfasst ein Bestimmen einer Ausgangsoberflächentemperatur in dem Hautbereich des Benutzers, Schritt 310. Das Verfahren 300 umfasst nach dem Bestimmen der Ausgangsoberflächentemperatur ein Verändern der Heizleistung des Infrarotstrahlers. Das Verändern erfolgt dabei derart, dass in einem Zeitraum von weniger als 5 Sekunden eine Differenz von wenigstens 20% in der den Benutzer abgegebenen Wärmeleistung in dem Hautbereich bewirkt ist, Schritt 320.

Das Verfahren 300 umfasst nach dem Verändern der Heizleistung das Erfassen einer Oberflächentemperatur in dem Hautbereich mittels wenigstens eines Wärmesensors, Schritt 330, sowie ein Bestimmen eines Temperaturverlaufs in dem Hautbereich des Benutzers wenigstens teilweise auf der Grundlage der erfassten Oberflächentemperatur mittels der Steuerungseinrichtung, wobei der Temperaturverlauf auf ein Wärmeabbauvermögen des Benutzers hinweist, Schritt 340.

Bei dem Verfahren 300 erfolgt zudem ein Verwenden des daraus ermittelten Wärmeabbauvermögens, beispielsweise eines Kennwerts, der auf das Wärmeabbauvermögen hinweist und der aus dem Temperaturverlauf durch Vergleichen mit einem gespeicherten Bezugstemperaturverlauf ermittelt ist, Schritt 350. Das Wärmeabbauvermögen wird dabei zum Bestimmen eine Regelspanne für eine Heizleistungsregelung der Wärmebehandlungsvorrichtung herangezogen, Teilschritt 352, und/oder wenigstens ein benutzerbezogener Kennwert, der auf das Wärmeabbauvermögen hinweist, wird mittels der Speichervorrichtung gespeichert, Teilschritt 354.

Die Erfindung ist voranstehend an konkreten Beispielen beschrieben. Es versteht sich jedoch, dass einige oder sämtliche Vorteile der Erfindung auch durch hiervon abweichende Umsetzungen der Erfindung erzielbar sind, beispielsweise durch Verwendung abweichender Anordnungen oder Anzahlen der beschriebenen Komponenten einer Wärmebehandlungsvorrichtung. Die Anzahlen und die Anordnungen der beschriebenen Komponenten sind dabei beispielsweise gemäß einem konkreten Anwendungsbedarf wählbar. Zudem versteht sich, dass Vorteile der Erfindung auch in anderen Anwendungsbereichen als der Wärmebehandlung von Menschen oder Tieren erzielbar sind. Dazu zählt beispielsweise die Wärmebehandlung von, insbesondere thermisch reaktiven, Werkstoffen und/oder Produkten.

## Patentansprüche

1. Wärmebehandlungsvorrichtung (100; 220) umfassend:
- wenigstens einen Infrarotstrahler (110, 112), der wenigstens eine steuerbare Wärmequelle umfasst und dazu angeordnet ist, Infrarotstrahlung, die mittels der Wärmequelle erzeugt ist, an einen Hautbereich eines Benutzers der Wärmebehandlungsvorrichtung (100; 200) auszusenden;
- wenigstens einen Wärmesensor (140, 142), der dazu angeordnet ist, eine Oberflächentemperatur in dem Hautbereich des Benutzers zu erfassen, und
- eine Steuerungseinrichtung (160), die dazu ausgebildet ist, eine Heizleistung des wenigstens einen Infrarotstrahlers (110, 112) wenigstens teilweise auf der Grundlage der erfassten Oberflächentemperatur in dem Hautbereich des Benutzers zu steuern,
wobei der Infrarotstrahler (110, 112) in einem Betriebszustand, der einer an den Benutzer abgegebenen Wärmeleistung im Bereich von 20 bis 120 Milliwatt pro Quadratzentimeter in dem Hautbereich des Benutzers entspricht, eine minimale Verzögerung von weniger als 5 Sekunden zum Erzielen einer Differenz von wenigstens 20% in der an den Benutzer in dem Hautbereich abgegebenen Wärmeleistung aufweist.

2. Wärmebehandlungsvorrichtung nach Anspruch 1, wobei die Wärmequelle ein keramikfreies wärmeerzeugendes Element umfasst.

3. Wärmebehandlungsvorrichtung nach Anspruch 1 oder 2, wobei die Wärmequelle eine steuerbare Halogenlampe, eine steuerbare Infrarot-, IR-, Leuchtdiode, LED, und/oder ein steuerbares Kohlefaser-Heizelement umfasst.

4. Wärmebehandlungsvorrichtung nach einem der vorhergehenden Ansprüche, wobei die Steuerungseinrichtung (160) dazu ausgebildet ist, die Heizleistung wenigstens teilweise im Rahmen einer Heizleistungsregelung auf der Grundlage der erfassten Oberflächentemperatur zu steuern.

5. Wärmebehandlungsvorrichtung nach Anspruch 4, wobei die Steuerungseinrichtung (160) dazu ausgebildet ist, die Wärmebehandlungsvorrichtung (100;220) zum Bestimmen eines benutzerspezifischen Wärmeabbauvermögens in dem Hautbereich des Benutzers zu steuern, und, nach einem Bestimmen des benutzerspezifischen Wärmeabbauvermögens, die Heizleistungsregelung gemäß einer Regelspanne, die wenigstens teilweise durch das benutzerspezifische Wärmeabbauvermögen bestimmt ist, durchzuführen.

6. Wärmebehandlungsvorrichtung nach Anspruch 5, wobei das Bestimmen des benutzerspezifischen Wärmeabbauvermögens umfasst:
- Bestimmen einer Ausgangsoberflächentemperatur in dem Hautbereich des Benutzers,
- Verändern einer Heizleistung des Infrarotstrahlers (110, 112) derart, dass in einem Zeitraum von weniger als 5 Sekunden eine Differenz von wenigstens 20% in der an den Benutzer abgegebenen Wärmeleistung in dem Hautbereich des Benutzers bewirkt ist, und
- Bestimmen eines Temperaturverlaufs in dem Hautbereich des Benutzers wenigstens teilweise auf der Grundlage einer Oberflächentemperatur in dem Hautbereich des Benutzers, die mittels des Wärmesensors (140, 142) nach dem Verändern der Heizleistung erfasst ist, wobei der Temperaturverlauf auf ein Wärmeabbauvermögen des Benutzers hinweist.

7. Wärmebehandlungsvorrichtung nach Anspruch 6, wobei:
das Bestimmen der Ausgangsoberflächentemperatur ein Erwärmen des Hautbereichs des Benutzers mittels Infrarotstrahlung, die mittels des Infrarotstrahlers (110, 112) erzeugt ist, zum Erreichen der Ausgangsoberflächentemperatur umfasst und das Verändern der Heizleistung ein Verringern der Heizleistung umfasst, wobei der Temperaturverlauf einem Abkühlen des Hautbereichs wenigstens teilweise infolge von Wärmeabbau im Körper des Benutzers entspricht, oder
das Verändern der Heizleistung ein Erhöhen der Heizleistung umfasst, wobei der Temperaturverlauf einem Aufwärmen des Hautbereichs entspricht, das wenigstens teilweise durch Wärmeabbau im Körper des Benutzers bestimmt ist.

8. Wärmebehandlungsvorrichtung nach einem der vorhergehenden Ansprüche, wobei die Steuerungseinrichtung (160) eine Speichervorrichtung (164) umfasst, die dazu ausgebildet ist, Programmcode zum Betreiben der Wärmebehandlungsvorrichtung (100; 220) gemäß wenigstens einem Wärmebehandlungsprogramm zu speichern, wobei, optional, die Speichervorrichtung (164) Programmcode zum Betreiben der Wärmebehandlungsvorrichtung (100; 220) gemäß wenigstens einem Wärmebehandlungsprogramm enthält, das während einer Wärmebehandlung in wenigstens einem Zeitabschnitt von weniger als 5 Sekunden eine Differenz von wenigstens 20% in der an den Benutzer abgegebenen Wärmeleistung in dem Hautbereich des Benutzers vorsieht.

9. Wärmebehandlungsvorrichtung nach einem der vorhergehenden Ansprüche, ferner umfassend wenigstens ein Spiegelelement (130, 132), das dazu ausgebildet ist, Infrarotstrahlung, die mittels des Infrarotstrahlers (110, 112) ausgesendet ist, in Richtung auf den Hautbereich des Benutzers umzulenken, wobei, optional, der Wärmesensor (140, 142) in einem Bereich des Spiegelelements (130, 132) angeordnet ist und, ferner optional, ein Erfassungsbereich des Wärmesensors (140, 142) sich wenigstens teilweise in einem Abschnitt eines Strahlungskegels, der durch die umgelenkte Infrarotstrahlung bestimmt ist, erstreckt.

10. Wärmebehandlungsvorrichtung nach Anspruch 9, wobei die Wärmebehandlungsvorrichtung (100; 220) wenigstens zwei Spiegelelemente (130, 132) und wenigstens zwei Infrarotstrahler (110, 112), die an verschiedenen Seiten in Bezug auf die Spiegelelemente (130, 132) angeordnet sind, umfasst, wobei, optional, die Wärmequelle jedes der Infrarotstrahler (110, 112) eine steuerbare Halogenröhre umfasst, die Halogenröhren zumindest im Wesentlichen parallel zueinander ausgerichtet sind, und eine Spiegelfläche jedes der Spiegelelemente (130, 132) zumindest im Wesentlichen parallel zu einer Längserstreckungsrichtung wenigstens einer der Halogenröhren ausgerichtet ist.

11. Wärmebehandlungsvorrichtung nach einem der vorhergehenden Ansprüche, wobei die Wärmebehandlungsvorrichtung (100; 220) eine Mehrzahl von Wärmesensoren (140, 142) umfasst, die dazu angeordnet sind, eine Oberflächentemperatur in jedem von verschiedenen Teilbereichen des Hautbereichs des Benutzers zu erfassen, wobei die Steuerungseinrichtung (160) dazu ausgebildet ist, die Heizleistung wenigstens teilweise auf der Grundlage der erfassten Oberflächentemperatur in den verschiedenen Teilbereichen des Hautbereichs des Benutzers zu steuern.

12. Wärmebehandlungsvorrichtung nach einem der vorhergehenden Ansprüche, wobei der Hautbereich des Benutzers eine Fläche von wenigstens 400 Quadratzentimetern aufweist und die Wärmebehandlungsvorrichtung (100; 220) derart ausgebildet ist, dass in einem Volumen, das sich ausgehend von dem Hautbereich und bis zu einem Abstand von wenigstens 10 Zentimetern von wenigstens einer Stelle des Hautbereichs parallel zu einer Einfallsrichtung der Infrarotstrahlung erstreckt, eine ortsabhängige Leistungsdichte der Infrarotstrahlung an jedweden verschiedenen Stellen des Volumens um höchstens 8% verschieden ist.

13. Wärmekabine (200) umfassend eine Wärmebehandlungsvorrichtung (100; 220) nach einem der vorhergehenden Ansprüche.

14. Wärmekabine nach Anspruch 13, wobei die Wärmekabine (200) ferner als Druckkammer, insbesondere zum Zweck hyperbarer Therapieanwendungen, ausgebildet ist.

15. Verfahren (300) zum benutzerbezogenen Kalibrieren einer Wärmebehandlungsvorrichtung (100; 220), wobei die Wärmebehandlungsvorrichtung (100; 220) umfasst:
- wenigstens einen Infrarotstrahler (110, 112), der wenigstens eine steuerbare Wärmequelle umfasst und dazu angeordnet ist, Infrarotstrahlung, die mittels der Wärmequelle erzeugt ist, an einen Hautbereich eines Benutzers der Wärmebehandlungsvorrichtung (100; 220) auszusenden;
- wenigstens einen Wärmesensor (140, 142), der dazu angeordnet ist, eine Oberflächentemperatur in dem Hautbereich des Benutzers zu erfassen, und
- eine Steuerungseinrichtung (160), die dazu ausgebildet ist, eine Heizleistung des wenigstens einen Infrarotstrahlers (110, 112) wenigstens teilweise auf der Grundlage der erfassten Oberflächentemperatur in dem Hautbereich des Benutzers zu steuern,
wobei das Verfahren (300) umfasst:
- Bestimmen einer Ausgangsoberflächentemperatur in dem Hautbereich des Benutzers;
- Verändern (320), nach dem Bestimmen der Ausgangsoberflächentemperatur (310) und mittels der Steuerungseinrichtung (160) und des Infrarotstrahlers (110, 112), einer Heizleistung des Infrarotstrahlers (110, 112) derart, dass in einem Zeitraum von weniger als 5 Sekunden eine Differenz von wenigstens 20% in der an den Benutzer abgegebenen Wärmeleistung in dem Hautbereich des Benutzers bewirkt ist;
- Erfassen (330), mittels des Wärmesensors (140, 142) und nach dem Verändern der Heizleistung, einer Oberflächentemperatur in dem Hautbereich des Benutzers;
- Bestimmen (340), mittels der Steuerungseinrichtung (160) und wenigstens teilweise auf der Grundlage der erfassten Oberflächentemperatur, eines Temperaturverlaufs in dem Hautbereich des Benutzers, wobei der Temperaturverlauf auf ein Wärmeabbauvermögen des Benutzers hinweist, und
- Bestimmen (350, 352), mittels der Steuerungseinrichtung (160) und wenigstens teilweise auf der Grundlage des Wärmeabbauvermögens des Benutzers, einer Regelspanne für eine Heizleistungsregelung der Wärmebehandlungsvorrichtung (100; 220), und/oder Speichern (350, 354), mittels einer Speichervorrichtung (164) der Steuerungseinrichtung (160), wenigstens eines benutzerbezogenen Kennwerts, der auf das Wärmeabbauvermögen des Benutzers hinweist.

## Claims

1. Heat treatment apparatus (100; 220) comprising:
at least one infrared radiator (110, 112) comprising at least one controllable heat source and arranged to emit infrared radiation generated by the heat source to a skin area of a user of the heat treatment apparatus (100; 200);
at least one thermal sensor (140, 142) arranged to detect a surface temperature at the skin area of the user; and
a control means (160) adapted to control a heat output of the at least one infrared radiator (110, 112) at least partially based on the detected surface temperature at the skin area of the user,
wherein the infrared radiator (110, 112) in an operating state corresponding to a heat output delivered to the user in the range of 20 to 120 milliwatts per square centimeter at the skin area of the user, has a minimum delay of less than 5 seconds to achieve a difference of at least 20% in the heat output delivered to the user at the skin area.

2. Heat treatment apparatus according to claim 1, wherein the heat source comprises a ceramic-free heat-generating element.

3. Heat treatment apparatus according to claim 1 or 2, wherein the heat source comprises a controllable halogen lamp, a controllable infrared, IR, light-emitting diode, LED, and/or a controllable carbon fiber heating element.

4. Heat treatment apparatus according to any one of the preceding claims, wherein the control means (160) is adapted to control the heat output at least in part as part of a heat output control based on the detected surface temperature.

5. Heat treatment apparatus according to claim 4, wherein the control means (160) is adapted to control the heat treatment apparatus (100; 220) to determine a user-specific heat dissipating capacity at the skin area of the user and, after determining the user-specific heat dissipating capacity, to carry out the heat output control in accordance with a control span that is determined at least in part by the user-specific heat dissipating capacity.

6. Heat treatment apparatus according to claim 5, wherein determining the user-specific heat dissipating capacity comprises:
determining an initial surface temperature in the skin area of the user;
varying a heat output of the infrared radiator (110, 112) so as to effect a difference of at least 20% in the heat output delivered to the user in the skin area of the user within a time period of less than 5 seconds; and
determining a temperature profile at the skin area of the user based at least in part on a surface temperature at the skin area of the user, which is detected by means of the heat sensor (140, 142) after the heat output has been changed, wherein the temperature profile indicates a heat dissipation capacity of the user.

7. Heat treatment apparatus according to claim 6, wherein:
determining the initial surface temperature comprises heating the skin area of the user by use of infrared radiation generated by means of the infrared radiator (110, 112) to attain the initial surface temperature, and varying the heat output comprises decreasing the heat output, wherein the temperature profile corresponds to a cooling of the skin area at least in part due to heat dissipation in the user's body, or
varying the heat output comprises increasing the heat output,
wherein the temperature profile corresponds to a heating of the skin area which is at least in part determined by heat dissipation in the user's body.

8. Heat treatment apparatus according to any one of the preceding claims, wherein the control means (160) includes a memory device (164) configured to store program code for operating the heat treatment apparatus (100; 220) according to at least one heat treatment program, wherein, optionally, the memory device (164) includes program code for operating the heat treatment apparatus (100; 220) according to at least one heat treatment program that provides, during a heat treatment, in at least one time segment of less than 5 seconds, a difference of at least 20% in the heat output delivered to the user at the skin area of the user.

9. Heat treatment apparatus according to any one of the preceding claims, further comprising at least one mirror element (130, 132) configured to redirect infrared radiation emitted by means of the infrared radiator (110, 112) toward the skin area of the user, wherein, optionally, the heat sensor (140, 142) is arranged in an area of the mirror element (130, 132) and, further optionally, a detection area of the heat sensor (140, 142) extends at least in part in a portion of a radiation cone that is determined by the redirected infrared radiation.

10. Heat treatment apparatus according to claim 9, wherein the heat treatment apparatus (100; 220) comprises at least two mirror elements (130, 132) and at least two infrared radiators (110, 112) arranged on different sides with respect to the mirror elements (130, 132), wherein optionally, the heat source of each of the infrared radiators (110, 112) comprises a controllable halogen tube, wherein the halogen tubes are aligned at least substantially parallel to one another, and wherein a mirror surface of each of the mirror elements (130, 132) is aligned at least substantially parallel to a longitudinal extension direction of at least one of the halogen tubes.

11. Heat treatment apparatus according to any one of the preceding claims, wherein the heat treatment apparatus (100; 220) comprises a plurality of heat sensors (140, 142) arranged to detect a surface temperature in each of different sub-areas of the skin area of the user, wherein the control means (160) is configured to control the heat output based at least in part on the detected surface temperature in the different sub-areas of the skin area of the user.

12. Heat treatment apparatus according to any one of the preceding claims, wherein the skin area of the user has a square measure of at least 400 square centimeters and the heat treatment apparatus (100; 220) is configured such that in a volume that extends from the skin area and up to a distance of at least 10 centimeters from at least one point of the skin area parallel to a direction of incidence of the infrared radiation, a location-dependent power density of the infrared radiation at any different points in the volume differs by at most 8%.

13. Heat cabin (200) comprising a heat treatment apparatus (100; 220) according to any one of the preceding claims.

14. Heat cabin according to claim 13, wherein the heat cabin (200) is further configured as a pressure chamber, in particular for the purpose of hyper-baric therapy applications.

15. Method (300) for user-related calibration of a heat treatment apparatus (100; 220), wherein the heat treatment apparatus (100; 220) comprises:
at least one infrared radiator (110, 112) comprising at least one controllable heat source and arranged to emit infrared radiation generated by means of the heat source to a skin area of a user of the heat treatment apparatus (100; 220);
at least one thermal sensor (140, 142) arranged to detect a surface temperature at the skin area of the user; and
a control means (160) configured to control a heat output of the at least one infrared radiator (110, 112) based at least in part on the detected surface temperature at the skin area of the user,
wherein the method (300) comprises the steps of:
determining an initial surface temperature at the skin area of the user;
changing, after determining the initial surface temperature (310) and by use of the control means (160) and the infrared radiator (110, 112), a heat output of the infrared radiator (110, 112) in such a way that in a period of less than 5 seconds a difference of at least 20% in the heat output delivered to the user at the skin area of the user is attained;
detecting (330), after changing the heat output, a surface temperature at the skin area of the user by means of the heat sensor (140, 142);
determining (340), by means of the control means (160) and based at least in part on the detected surface temperature, a temperature profile at the skin area of the user, wherein the temperature profile indicates a user's heat dissipation capacity; and
determining (350, 352), by means of the control means (160) and at least in part based on the user's heat dissipation capacity, a control span for a heat output control of the heat treatment apparatus (100; 220), and/or storing (350, 354), by means of a memory device (164) of the control means (160), at least one user-related characteristic value that indicates the user's heat dissipation capacity.

## Revendications

1. Dispositif de traitement thermique (100 ; 220) comprenant :
- au moins un émetteur infrarouge (110, 112), qui comprend au moins une source de chaleur contrôlable et conçue pour émettre un rayonnement infrarouge, qui est généré par la source de chaleur, vers une zone cutanée d'un utilisateur du dispositif de traitement thermique (100 ; 200 );
- au moins un capteur thermique (140, 142) agencé pour détecter une température de surface dans la zone cutanée de l'utilisateur, et
- un dispositif de commande (160) qui est conçu pour commander une puissance de chauffage du au moins un émetteur infrarouge (110, 112) au moins en partie sur la base de la température de surface détectée dans la zone cutanée de l'utilisateur,
dans lequel l'émetteur infrarouge (110, 112), dans un état de fonctionnement qui correspond à une puissance calorifique délivrée à l'utilisateur dans la plage de 20 à 120 milliwatts par centimètre carré dans la zone cutanée de l'utilisateur, présente un délai minimum de moins de 5 secondes pour obtenir une différence d'au moins 20 % dans la puissance calorifique transmise à l'utilisateur au niveau de la zone cutanée.

2. Dispositif de traitement thermique selon la revendication 1, dans lequel la source de chaleur comprend un élément générateur de chaleur sans céramique.

3. Dispositif de traitement thermique selon la revendication 1 ou 2, dans lequel la source de chaleur comprend une lampe halogène contrôlable, une diode électroluminescente infrarouge, IR, LED contrôlable, et/ou un élément chauffant contrôlable en fibre de carbone.

4. Dispositif de traitement thermique selon l'une quelconque des revendications précédentes, dans lequel le dispositif de commande (160) est conçu pour contrôler la puissance de chauffage au moins partiellement dans le cadre d'un contrôle de la puissance de chauffage basé sur la température de surface détectée.

5. Dispositif de traitement thermique selon la revendication 4, dans lequel le dispositif de commande (160) est conçu pour contrôler le dispositif de traitement thermique (100 ; 220) pour déterminer une capacité de dissipation thermique spécifique à l'utilisateur dans la zone cutanée de l'utilisateur, et après avoir déterminé la capacité de dissipation thermique spécifique à l'utilisateur pour effectuer la commande de la puissance de chauffage selon une plage de commande déterminée au moins en partie par la capacité de dissipation thermique spécifique à l'utilisateur.

6. Dispositif de traitement thermique selon la revendication 5, dans lequel la détermination de la capacité de dissipation thermique spécifique à l'utilisateur comprend :
- déterminer une température de surface initiale dans la zone cutanée de l'utilisateur,
- modifier une puissance de chauffage de l'émetteur infrarouge (110, 112) de telle sorte que, dans un laps de temps inférieur à 5 secondes, il se produise une différence d'au moins 20 % dans la puissance thermique délivrée à l'utilisateur dans la zone cutanée de l'utilisateur, et
- déterminer un profil de température dans la zone cutanée de l'utilisateur au moins partiellement basée sur une température de surface dans la zone cutanée de l'utilisateur, qui est détecté au moyen du capteur thermique (140, 142) après modification de la puissance de chauffage, le profil de température étant indicatif d'une capacité de dissipation thermique de l'utilisateur.

7. Dispositif de traitement thermique selon la revendication 6, dans lequel : la détermination de la température de surface initiale comprend le chauffage de la zone cutanée de l'utilisateur au moyen d'un rayonnement infrarouge généré au moyen de l'émetteur infrarouge (110, 112), pour atteindre la température de surface initiale et la modification de la puissance de chauffage comprend la réduction de la puissance de chauffage, l'évolution de la température correspondant à un refroidissement de la zone cutanée au moins partiellement en raison d'une perte de chaleur dans le corps de l'utilisateur, ou la modification de la puissance de chauffage comprend une augmentation de la puissance de chauffage, l'évolution de la température correspondant à un réchauffement de la zone cutanée, qui est au moins partiellement déterminé par la perte de chaleur dans le corps de l'utilisateur.

8. Dispositif de traitement thermique selon l'une quelconque des revendications précédentes, dans lequel le dispositif de commande (160) comprend un dispositif mémoire (164) qui est conçu pour stocker un code de programme pour faire fonctionner le dispositif de traitement thermique (100 ; 220) selon au moins un programme de traitement thermique, dans lequel, facultativement, le dispositif mémoire (164) contient un code de programme pour faire fonctionner le dispositif de traitement thermique (100 ; 220) selon au moins un programme de traitement thermique qui, pendant un traitement thermique, produit une différence d'au moins 20 % de la puissance calorifique délivrée à l'utilisateur pendant au moins une période de temps inférieure à 5 secondes sur la zone cutanée de l'utilisateur.

9. Dispositif de traitement thermique selon l'une quelconque des revendications précédentes, comprenant en outre au moins un élément miroir (130, 132), conçu pour rediriger le rayonnement infrarouge émis au moyen de l'émetteur infrarouge (110, 112) vers la zone cutanée de l'utilisateur, dans lequel, facultativement, le capteur thermique (140, 142) est disposé dans une région de l'élément miroir (130, 132) et, en outre facultativement, une zone de détection du capteur thermique (140, 142) s'étend au moins partiellement dans une partie d'un cône de rayonnement déterminé par le rayonnement infrarouge redirigé.

10. Dispositif de traitement thermique selon la revendication 9, dans lequel le dispositif de traitement thermique (100 ; 220) comporte au moins deux éléments miroirs (130, 132) et au moins deux émetteurs infrarouges (110, 112) disposés sur des côtés différents par rapport aux éléments miroirs (130, 132), dans lequel, facultativement, la source de chaleur de chacun des émetteurs infrarouges (110, 112) comprend un tube halogène contrôlable, les tubes halogènes sont alignés au moins sensiblement parallèlement les uns aux autres, et une surface de miroir de chacun des éléments miroirs (130, 132) est au moins sensiblement parallèle à une direction longitudinale d'au moins un des tubes halogènes.

11. Dispositif de traitement thermique selon l'une quelconque des revendications précédentes, dans lequel le dispositif de traitement thermique (100 ; 220) comprend une pluralité de capteurs thermiques (140, 142) agencés pour détecter une température de surface dans chacune des différentes parties de la zone cutanée de l'utilisateur, dans lequel le dispositif de commande (160) est conçu pour contrôler la puissance de chauffage au moins partiellement sur la base de la température de surface détectée dans les différentes sous-zones cutanées de l'utilisateur.

12. Dispositif de traitement thermique selon l'une quelconque des revendications précédentes, dans lequel la zone cutanée de l'utilisateur a une superficie d'au moins 400 centimètres carrés et le dispositif de traitement thermique (100 ; 220) est conçu de telle sorte que dans un volume qui s'étend à partir de la zone cutanée et jusqu'à une distance d'au moins 10 centimètres à partir d'au moins un point de la zone cutanée parallèlement à une direction d'incidence du rayonnement infrarouge, une densité de puissance du rayonnement infrarouge dépendant de l'emplacement diffère d'un maximum de 8 % en différents points du volume.

13. Cabine chauffante (200) comprenant un dispositif de traitement thermique (100 ; 220) selon l'une des revendications précédentes.

14. Cabine chauffante selon la revendication 13, dans laquelle la cabine thermique (200) est en outre conçue comme une chambre de pression, notamment en vue d'applications de thérapie hyperbare.

15. Procédé (300) d'étalonnage d'un dispositif de traitement thermique (100 ; 220) en fonction de l'utilisateur, le dispositif de traitement thermique (100 ; 220) comprenant :
- au moins un émetteur infrarouge (110, 112), qui comprend au moins une source de chaleur contrôlable et agencé pour émettre un rayonnement infrarouge, qui est généré par la source de chaleur, vers une zone cutanée d'un utilisateur du dispositif de traitement thermique (100 ; 220);
- au moins un capteur thermique (140, 142) agencé pour détecter une température de surface dans la zone cutanée de l'utilisateur, et
- un dispositif de commande (160) qui est conçu pour commander une puissance de chauffage du au moins un émetteur infrarouge (110, 112) au moins en partie sur la base de la température de surface détectée dans la zone cutanée de l'utilisateur,
dans lequel le procédé (300) comprend :
- déterminer une température de surface initiale dans la zone cutanée de l'utilisateur ;
- modifier (320), après détermination de la température de surface initiale (310) et au moyen du dispositif de commande (160) et de l'émetteur infrarouge (110, 112), une puissance de chauffage de l'émetteur infrarouge (110, 112) de telle sorte qu'en une période de moins de 5 secondes, une différence d'au moins 20 % dans la puissance thermique délivrée à l'utilisateur soit provoquée dans la zone cutanée de l'utilisateur ;
- détecter (330), au moyen du capteur thermique (140, 142) et après modification de la puissance de chauffage, une température de surface au niveau de la peau de l'utilisateur ;
- déterminer (340), au moyen du dispositif de commande (160) et au moins partiellement sur la base de la température de surface détectée, un profil de température au niveau de la zone cutanée de l'utilisateur, le profil de température étant indicative d'une capacité de l'utilisateur à dissiper la chaleur, et
- déterminer (350, 352), au moyen du dispositif de commande (160) et au moins en partie en fonction de la capacité de dissipation thermique de l'utilisateur, une plage de contrôle pour le contrôle de la puissance de chauffage du dispositif de traitement thermique (100 ; 220), et /ou stocker (350, 354), au moyen d'un dispositif mémoire (164) du dispositif de commande (160), au moins une valeur caractéristique relative à l'utilisateur qui est indicative de la capacité de dissipation thermique de l'utilisateur.
